# EUROPEAN PATENT APPLICATION

(11) **EP 4 328 362 A1**
(43) Date of publication of application: **28.02.2024**
(21) Application number: 21937340.4
(22) Date of filing: 22.04.2021
(51) Int. Cl.: C40B 50/06, C12Q 1/68, C12N 15/10

(54) **CONSTRUCTION METHOD FOR RNA SEQUENCING LIBRARY, SEQUENCING METHOD, AND KIT**

(71) Applicant: BGI Shenzhen, Shenzhen, Guangdong 518083 (CN)
(72) Inventor: LIU, Longqi, Shenzhen, Guangdong 518083 (CN); LIN, Xiumei, Shenzhen, Guangdong 518083 (CN); SHI, Quan, Shenzhen, Guangdong 518083 (CN); SHI, Xuyang, Shenzhen, Guangdong 518083 (CN); LIU, Chuanyu, Shenzhen, Guangdong 518083 (CN); HUANG, Yaling, Shenzhen, Guangdong 518083 (CN); LIU, Ya, Shenzhen, Guangdong 518083 (CN)
(74) Representative: Novagraaf Technologies
(86) International application number: PCT/CN2021/088984
(87) International publication number: WO 2022/222101

(57) **Abstract**

Provided in the present application is a method for constructing an RNA sequencing library, a sequencing method and a kit. The construction method comprises: acquiring a single-stranded cDNA, which is a reverse transcription product of mRNA, the 3'-terminal of the single-stranded cDNA including a cDNA tag sequence; cyclizing the single-stranded cDNA to obtain a single-stranded cyclized cDNA; amplifying the single-stranded cyclized cDNA with a primer combination, which is formed by a random primer or a gene-specific primer and a cDNA tag primer, so as to obtain an amplified fragment, the cDNA tag primer being at least a part of the cDNA tag sequence; and performing fragmentation for library construction on the amplified fragment, so as to obtain an RNA sequencing library.

## Description

### Technical Field

The present invention relates to the field of sequencing, and specifically, to a method for constructing an RNA sequencing library, a sequencing method and a kit.

### Background

With the rapid development of a single-cell technology, the era of single-cell sequencing was soon ushered in. The single-cell technology has also been updated and iterated. From single-tube amplification to the current droplet-based high throughput, there have been great changes in terms of labor, time, cost, and cell capture. However, due to the limitation of the read length of sequencing, full-length cDNA often needs to be interrupted to construct a library for sequencing. A Smart-seq2 technology uses a single-tube amplification strategy, and then uses technologies, such as flow cytometry and microdissection, to divide single cells into corresponding single wells for lysis, transcription and amplification, interruption, and library construction. Since there is no specific barcode to distinguish cell sources for single tube library construction, all sequences of a cell can only be tagged during library construction, and finally libraries from different cell sources are combined for sequencing. Such a sequencing strategy obtains the full length, but the throughput of the cells is limited, and the cost of sequencing is also greatly increased.

For the current droplet-based high-throughput sequencing strategy, sequences at both terminals of cDNA are specifically captured by synthesizing a sequence carrying a specific molecular marker on beads. However, during the limitation of the read length of sequencing, sequencing cannot be directly performed on the full-length cDNA carrying a tag, and further interruption is still required. Since the sequence in the middle of the cDNA sequence after interruption is not tagged and filtered out, there is no way to obtain the full length, thereby losing a lot of important information such as variable shear. The droplet-based high-throughput sequencing strategy includes the following specific steps.

On the basis of a droplet microfluidic technology, a water-in-oil droplet is used to wrap a cell and a bead to obtain a droplet simultaneously including the cell and the bead. Other components in the droplet further include a lysis buffer, and the bead includes a large number of DNA sequences with specific molecular markers (barcodes), and a TSO sequence. During the continuous generation of the droplet, the cell inside the droplet is lysed by the lysis buffer, so as to release a large amount of mRNA. At the same time, the mRNA is captured by free poly(dT), and an RT reaction is completed in the droplet. In addition, the TSO sequence is added to the terminal of the mRNA. A complementary sequence of TSO on the extended terminal of first strand cDNA binds to the TSO on the bead, such that the mRNA is captured onto the bead. By means of PCR amplification, a 5' sequence is tagged with a specific molecular marker, so as to achieve single-cell high-throughput sequencing of 5' RNA, for example, 10x Genomics. However, the bigger problem with the 10x Genomics at present is the high cost.

The main companies offering single-cell sequencing instruments and services in the market are 10X Genomics, BD, and Nadia of Dolomite. Except for the 10X Genomics, there is a lack of methods for high-throughput 5'RNA sequencing, and 5'RNA seq is an essential part for the acquisition of immunome libraries, for example, 5'TCR/BCR is an important component of immune cells, it is crucial to obtain a TCR/BCR sequence to gain insight into immune mechanisms. In addition, droplet-based high-throughput single-cell sequencing of full-length mRNA has also not yet been reported, and full length plays an important role in understanding of gene diversity and regulatory mechanisms.

From the above, it can be learned that the current full-length RNA sequencing based on single tubes are difficult to achieve high-throughput single-cell sequencing. The shortcoming of droplet microfluidics is that since the molecular marker can only be designed at the 3' terminal or 5' terminal of the cDNA, after being interrupted and screened during library construction, an intermediate sequence is discarded because there is no specific molecular marker to trace the origin of the fragment, and therefore the full length cannot be obtained. In addition, although 10x Genomics currently achieves single-cell high-throughput sequencing of the 5' RNA, TSO-based complementary capture of mRNA is not as efficient as poly(dT)-based capture and is also more costly.

Therefore, improvements to existing methods are still needed to achieve high-throughput sequencing of the RNA 5' ends or full length of RNA in the single cell.

### Summary

The present invention is mainly intended to provide a method for constructing an RNA sequencing library, a sequencing method and a kit, to solve the problem in the prior art that it is difficult to achieve high-throughput sequencing of the 5' terminal or full length of RNA.

In order to achieve the above objective, an aspect of the present invention provides a method for constructing an RNA sequencing library. The method includes: acquiring a single-stranded cDNA, which is a reverse transcription product of mRNA, where the 3'-terminal of the single-stranded cDNA includes a cDNA tag sequence; cyclizing the single-stranded cDNA to obtain a single-stranded cyclized cDNA; amplifying the single-stranded cyclized cDNA with a primer combination, which is formed by a random primer or a gene-specific primer and a cDNA tag primer, so as to obtain an amplified fragment, where the cDNA tag primer is at least a part of the cDNA tag sequence; and performing fragmentation for library construction on the amplified fragment, so as to obtain the RNA sequencing library.

Further, the operation of acquiring the single-stranded cDNA, which is the reverse transcription product of mRNA, where the 3'-terminal of the single-stranded cDNA includes the cDNA tag sequence includes: performing reverse transcription on the mRNA, so as to obtain a first strand cDNA; amplifying the first strand cDNA to obtain a double-stranded cDNA, where the 3' terminal of a second strand cDNA, which is complementary to the first strand cDNA includes the cDNA tag sequence, and the cDNA tag sequence comprises a poly(A); and melting the double-stranded cDNA to obtain the single-stranded cDNA.

Further, the cDNA tag sequence successively includes, in a direction from 3' to 5', a second PCR adapter, a second cell barcode, a second Unique Molecular Identifier (UMI) and the poly(A).

Further, the mRNA is derived from a single-cell sample, and the mRNA is a single-cell mRNA.

Further, preparing the single-cell mRNA with a droplet method, so as to make the single-cell mRNA ligated to a solid support, and preferably, the solid support is a bead.

Further, the operation of preparing the single-cell mRNA with the droplet method, so as to make the single-cell mRNA ligated to the bead includes: respectively providing one single-cell suspension and the bead, where the bead carries a bead tag sequence, and the terminal of the bead tag sequence includes poly(dT); and wrapping the single-cell suspension and the bead into droplets, where each droplet includes a single cell and one bead, and the bead is combined with the poly(A) of the mRNA in the single-cell suspension through the poly(dT), to connect the mRNA in the single-cell suspension to the bead, so as to obtain the single-cell mRNA.

Further, the bead tag sequence successively includes, in a direction from 5' to 3', a first PCR adapter, a first cell barcode, a first UMI and the poly(dT); and correspondingly, the cDNA tag sequence successively includes, in a direction from 3' to 5', a second PCR adapter, a second cell barcode, a second UMI and a poly(A). The second PCR adapter is complementary to the first PCR adapter; the second cell barcode is complementary to the first cell barcode; and the second UMI is complementary to the first UMI.

Further, the 5' terminal of the single-stranded cDNA includes a sequence of a TSO primer.

Further, reverse transcription is performed on the mRNA with a reverse transcriptase and a TSO adapter, so as to obtain the first strand cDNA. The reverse transcriptase has a terminal transferase activity, and the 3' terminal of the first strand cDNA includes a complementary sequence of the TSO adapter; and the first strand cDNA is amplified to obtain the second strand cDNA, and the 5' terminal of the second strand cDNA includes the sequence of the TSO primer.

Further, the sequence of the TSO adapter is SEQ ID NO: 1.

Further, the reverse transcriptase is selected from an Alpha reverse transcriptase of MGI, a Superscript^{™}II reverse transcriptase of Invitrogen, Superscript IV of Thermo, or Maxima H Minus of Thermo.

Further, random amplification and/or full-length amplification is performed on the first strand cDNA, so as to obtain the double-stranded cDNA.

Further, the first strand cDNA is amplified with an adapter amplification primer and a TSO primer, so as to obtain the double-stranded cDNA; or the first strand cDNA is amplified with the adapter amplification primer, a TSO-random primer and the TSO primer, so as to obtain the double-stranded cDNA.

Further, the sequence of the adapter amplification primer is SEQ ID NO: 2; the sequence of the TSO primer is SEQ ID NO: 3; and the sequence of the TSO-random primer is SEQ ID NO: 4.

Further, the operation of cyclizing the single-stranded cDNA to obtain the single-stranded cyclized cDNA includes: ligating the single-stranded cDNA into a ring under the action of a cyclization auxiliary sequence and a ligase, so as to obtain a ligated product; and performing enzyme digestion on the ligated product to digest the single-stranded cDNA, which is not ligated into the ring, so as to obtain the single-stranded cyclized cDNA. The cyclization auxiliary sequence is complementary to sequences on two terminals of the single-stranded cDNA.

Further, the cyclization auxiliary sequence is selected from SEQ ID NO: 5.

Further, the gene-specific primer is a TCR primer for TCR gene amplification and/or a BCR primer for BCR gene amplification.

Further, the cDNA tag primer is a poly(A) primer, preferably SEQ ID NO: 6.

Further, the operation of performing fragmentation for library construction on the amplified fragment, so as to obtain the RNA sequencing library includes: adding a library adapter to the amplified fragment, so as to obtain the RNA sequencing library.

Further, fragmentation with enzyme digestion is performed on the amplified fragment, so as to obtain digested fragments; and terminal repair, A tailing addition and library adapter ligation are successively performed on the digested fragments, so as to obtain the RNA sequencing library.

Further, after library adapter ligation is performed, the method further includes performing PCR amplification on the ligated product of the library adapter, so as to obtain the RNA sequencing library.

Further, the library adapter is an adapter of an MGI sequencing platform or an adapter of an Illumina sequencing platform.

A second aspect of the present application provides a kit for an RNA library construction. The kit includes: a cyclization auxiliary sequence, a DNA ligase, a cDNA tag primer, and at least one of the following primers: (a) a random primer; (b) a TCR primer; or (c) a BCR primer.

Further, the kit further includes an RNA reverse transcription reagent.

Further, the RNA reverse transcription reagent includes a reverse transcriptase; and the reverse transcriptase is a reverse transcriptase having terminal transferase activity.

Further, the reverse transcriptase is selected from an Alpha reverse transcriptase of MGI, a Superscript^{™}II reverse transcriptase of Invitrogen, Superscript IV of Thermo, or Maxima H Minus of Thermo.

Further, the RNA reverse transcription reagent further includes a TSO adapter.

Further, the sequence of the TSO adapter is SEQ ID NO: 1.

Further, the kit further includes a TSO primer and an adapter amplification primer.

Further, the sequence of the adapter amplification primer is SEQ ID NO: 2; and the sequence of the TSO primer is SEQ ID NO: 3.

Further, the kit further includes a TSO-random primer.

Further, the sequence of the TSO-random primer is SEQ ID NO: 4.

Further, the cyclization auxiliary sequence is SEQ ID NO: 5.

Further, the cDNA tag primer is a poly(A) primer.

Further, the sequence of the cDNA tag primer is SEQ ID NO: 6.

Further, the kit further includes at least one of an exonuclease or a library adapter.

Further, the exonuclease is selected from an exonuclease I or exonuclease III.

Further, the library adapter is an adapter of an MGI sequencing platform or an adapter of an Illumina sequencing platform.

Further, the MGI sequencing platform is selected from a bubble adapter; and an adapter of the Illumina sequencing platform is selected from P5 and P7 adapters.

Further, the DNA ligase is selected from a T4 DNA ligase.

Further, the kit further includes a solid support. The solid support is provided with a support tag sequence. The cDNA tag primer is complementary to at least a part of the support tag sequence. Preferably, the solid support is a bead, and the support tag sequence is a bead tag sequence.

Further, the support tag sequence successively includes, in a direction from 5' to 3', a first PCR adapter, a first cell barcode, a first UMI and the poly(dT).

A third aspect of the present application provides A method for sequencing an RNA library. The method includes: constructing an RNA sequencing library with any one of the foregoing methods for constructing an RNA sequencing library, and performing sequencing on the RNA sequencing library.

By means of the technical solutions of the present invention, the single-stranded cDNA, which is a reverse transcription product of mRNA, is obtained, and the 3'-terminal of the single-stranded cDNA includes the cDNA tag sequence; the single-stranded cDNA with the cDNA tag sequence is cyclized to ligate two terminals of the single-stranded cDNA, which corresponding to ligate the 5' terminal and the 3' terminal of the mRNA, such that a fragment of the 5' terminal of the mRNA is tagged by means of a 3'-terminal cDNA tag sequence. Then, by means of amplifying single-stranded cyclized cDNA with the cDNA tag primer, which is the same as at least a part of the cDNA tag sequence, and the random primer or the gene-specific primer, an amplified fragment starting from the position of a specific gene or any position at the 5' terminal is obtained; and finally, fragmentation selection is performed on these amplified fragments, and then library construction and sequencing are performed, such that the purpose of high-throughput sequencing is achieved. Therefore, on the basis of whether the length of the obtained single-stranded cDNA used for cyclization is that of a full-length cDNA or a random-length cDNA, a 5' RNA sequencing library, or a full-length RNA sequencing library may be obtained according to different research purposes.

### Brief Description of the Drawings

The drawings, which form a part of this application, are used to provide a further understanding of the present invention. The exemplary embodiments of the present invention and the description thereof are used to explain the present invention, but do not constitute improper limitations to the present invention. In the drawings:
Fig. 1 is a schematic structural diagram of a chip for preparing a droplet according to Embodiment 1 of the present invention.
Fig. 2 is a schematic flowchart of an RNA full-length library construction principle and library construction and sequencing thereof according to the present invention.
Fig. 3 is a schematic flowchart of a 5' RNA terminal library construction principle and library construction and sequencing thereof according to the present invention.
Fig. 4A and Fig. 4B show detection results of an Agilent 2100 biological analyzer of full-length cDNA amplification products of a cell line sample and a solid tissue sample according to Embodiment 1 of the present invention.
Fig. 5A and Fig. 5B show detection results of an Agilent 2100 biological analyzer of random primer amplified cDNA products of a cell line sample and a solid tissue sample according to Embodiment 2 of the present invention.
Fig. 6A and Fig. 6B show detection results of an Agilent 2100 biological analyzer of random primer amplified products of a cell line sample and a solid tissue sample, after a single-stranded cDNA is cyclized, according to Embodiment 1 of the present invention.
Fig. 7A and Fig. 7B show detection results of an Agilent 2100 biological analyzer of TCR/BCR primer amplified products of a cell line sample and a solid tissue sample, after a single-stranded cDNA is cyclized, according to Embodiment 2 of the present invention.
Fig. 8 shows analysis results of the coverage of transcripts by 5' terminal and 3' terminal sequencing fragments in sequencing data, after sequencing analysis of the library constructed according to a preferred embodiment of the present application.

### Detailed Description of the Embodiments

It is to be noted that the embodiments in this application and the features in the embodiments may be combined with one another without conflict. The present invention will be described below in detail with reference to the embodiments.

### Explanation of terms:

Template Switch Oligo (TSO) is sometimes called "a TSO adapter" in the present application, which is used in conjunction with a reverse transcriptase having terminal transferase activity. When performing reverse transcription on mRNA, the reverse transcriptase having terminal transferase activity adds CCC (only to a full-length transcript) to the terminal of a first strand cDNA. The TSO adapter carries rGrG+G (rG represents ribose guanine nucleotide, and +G represents LNA-modified deoxyribose guanine nucleotide) paired and combined with the CCC, such that during reverse transcription, the 3' terminal CCC of the first strand cDNA carries a complementary sequence of the TSO adapter other than rGrG+G.

TSO primer may be at least partially combined with the 3' terminal of the first strand cDNA, and is used for synthesis of a second strand cDNA and cDNA amplification. In a specific implementation, the TSO primer is a sequence of the TSO adapter with rGrG+G removed.

Random primer and TSO-random primer: the random primer in the present application refers to a sequence which only consists of base N, for example, a random sequence consisting of N of 6-12 nt; and the TSO-random primer refers to a sequence including the TSO primer at the upstream of the random primer consisting of base N.

Support tag sequence: in the present application, referring to a tag sequence ligated to a solid support for capturing the mRNA, and at least including oligo(dT) for capturing the mRNA. In some embodiments, the support tag sequence includes a cell barcode and the oligo(dT) in the order of 5' to 3'. The oligo(dT) is used for being complementary to poly(A) of the mRNA, so as to capture the mRNA; and the cell barcode is used for tagging the mRNA from the same cell. In some embodiments, in order to further tag different mRNA molecules in the same cell, a UMI may be arranged between the cell barcode and the oligo(dT). In some preferred embodiments, for ease of subsequent library construction, a PCR adapter may further be arranged in a 5' direction of the cell barcode for subsequent PCR amplification. In some preferred embodiments, the solid support is a bead. A tag sequence on the bead is recorded as a bead tag sequence, and successively includes, according to a sequence from near to far of the bead (that is, a sequence from 5' to 3'): a PCR adapter, the cell barcode, the UMI and the oligo(dT).

cDNA tag sequence: a tag sequence on the cyclized single-stranded cDNA with the poly(A), where the poly(A) is used for being complementary to the oligo(dT) on the solid support, such that the mRNA captured by the solid support is amplified. In some preferred embodiments, in addition to including the poly(A), the cDNA tag sequence further includes the cell barcode for tagging a cell source. In some referred embodiments, the UMI is also arranged between the cell barcode and the oligo(dT), so as to tag different mRNA molecules in the same cell. In some other preferred embodiments, a PCR adapter is also arranged in a 3' direction of the cell barcode, so as to be used as a primer for PCR amplification in the subsequent library construction step. In a more preferred embodiment, the cDNA tag sequence successively includes, according to a sequence from 3' to 5', a PCR adapter, a cell barcode, a UMI and the poly(A). In the present application, in order to distinguish sequences more accurately, the PCR adapter, the cell barcode and the UMI on the support tag sequence (or the bead tag sequence) are respectively recorded as a first PCR adapter, a first cell barcode, a first UMI. Correspondingly, the PCR adapter, the cell barcode and the UMI on the cDNA tag sequence are respectively recorded as a second PCR adapter, a second cell barcode, a second UMI.

Adapter amplification primer: the adapter amplification primer in the present application is the PCR adapter, which is described from the perspective of cDNA amplification; and the adapter amplification primer and the TSO primer are used as a primer combination for amplification.

cDNA tag primer: referring to a primer used for amplification of the cyclized single-stranded cDNA, and is at least a part of the tag sequence ligated to the poly(A) of the cyclized single-stranded cDNA, for example, may be the poly(A) or the cDNA tag sequence, and is used for amplifying the sequence with poly(T) after amplification of the cyclized single-stranded cDNA by the random primer or the gene-specific primer, so as to obtain an amplified fragment required to construct a 5' RNA library or a full-length RNA library.

TCR/BCR primer: TCR/BCR refers to T Cell Receptor/B Cell Receptor, that is, a primer encoding a T cell receptor or B cell receptor gene, and is one of the gene-specific primers used for single-cell T/B cell receptor sequencing, so as to study an immune mechanism.

Auxiliary cyclization sequence: in the present application, referring to an auxiliary sequence, which is used for bringing the 5' terminal and 3' terminal of the single-stranded cDNA close to each other to achieve the proximity from end to end during the cyclization of the single-stranded cDNA. Sequences, which are respectively complementary to the 5' terminal and the 3' terminal of the single-stranded cDNA, are used to bring the 5' terminal and the 3' terminal of the single-stranded cDNA close to each other; there may be a gap between the 5' terminal and the 3' terminal, which close to each other; and the gap is filled under the action of a DNA ligase, so as to achieve the cyclization of the single-stranded cDNA.

poly(A): it is known to those skilled in the art that the mRNA has a poly(A) tail (polyadenylate), and accordingly, the poly(A) tail corresponding to DNA in the present application refers to polydeoxyadenylate, which is complementarily paired with the poly(dT).

As mentioned in the Background, an existing RNA sequencing method that can sequence full-length mRNA is difficult to achieve high throughput, and most of the methods that can achieve high throughput sequencing target mRNA sequencing at the 3' terminal. Single-cell sequencing at the 5' terminal is currently dominated by 10x Genomics, while sequencing of single-cell full-length mRNA has not been reported. In order to improve this situation, the inventors according to the shortcomings of the existing single-cell sequencing technology to improve the existing single-cell RNA library construction method, and after finding that there is no report on capturing cDNA by means of 3' terminal RNA based single-cell high-throughput sequencing, transfer a tag sequence at the 3' terminal of the mRNA to the 5' terminal of the mRNA by means of cyclization ligation, or shares a tag with the 3' terminal, so as to simultaneously achieve RNA single-cell high-throughput sequencing at the 3' terminal and/or 5' terminal. Specifically, the inventors carry out a detailed study respectively on the construction of a full-length cDNA single-cell high-throughput sequencing library and the construction of an RNA single-cell high-throughput sequencing library at the 5' terminal, and further refines the experimental design to confirm the feasibility of the method. Specific principles and steps respectively include the following.

### (I) RNA full-length single-cell sequencing strategy

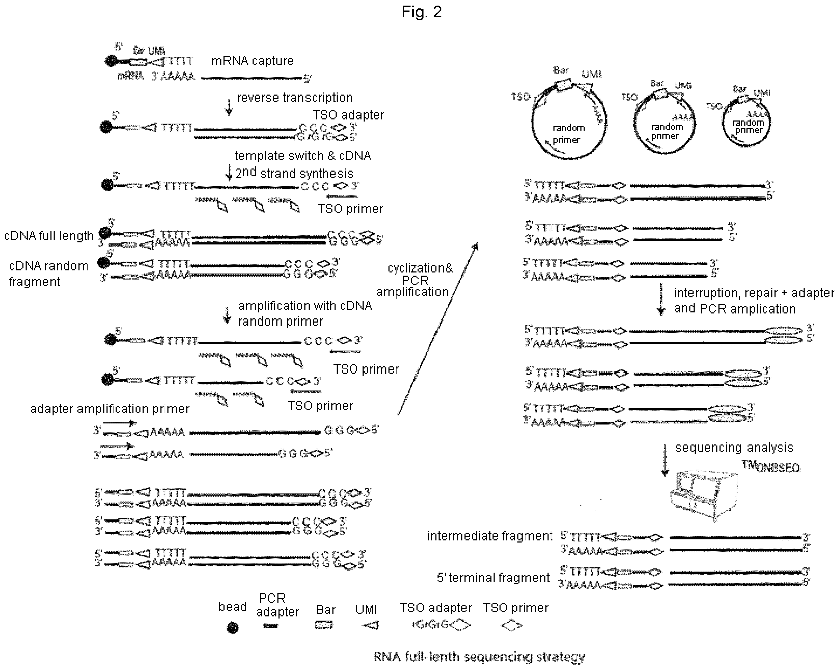

The mRNA is captured on the basis of a 3' terminal RNA_seq droplet strategy, and reverse transcription is performed to generate a cDNA full length. Then, cDNA is amplified with the adapter amplification primer, the TSO primer and the TSO-random primer. The bead tag sequence at the 3' terminal and from the solid support such as the bead is converted into the cDNA tag sequence for reservation as the bead tag sequence is amplified; and the 5' terminal form DNA fragments with variable lengths according to different positions at which the TSO-random primer is combined, such that the fragments with different lengths are ligated and cyclized, so as to form an end-to-end ring of the cDNA tag sequence at the 3' terminal and the TSO primer at the 5' terminal. Then, amplification is performed by means of the random primer complementary to a cDNA ring and a poly(A) primer by further using the ring as a template; and these amplified products are fragmented and screened, and then library construction and sequencing is performed, so as to not only amplify the cDNA full length but also achieve the purpose of high-throughput sequencing (as shown in Fig. 2).

### Specific steps include the following.

(1) A segment of PCR adapter sequence of poly(A) at the 3' terminal that is used for capturing the mRNA is designed. The PCR adapter sequence may be ligated to the solid support (for example, the bead); the PCR adapter may be used as an adapter amplification primer during cDNA amplification; a segment of TSO primer complementary to the 3' terminal of a first strand cDNA, and a TSO-random primer are designed; after the synthesis of the first strand cDNA, amplification is performed with the above primers, so as to obtain cDNA fragments with different sizes.
(2) Through cyclization ligation, a single-stranded 3' terminal cDNA tag sequence with the poly(A) in the cDNA fragments is ligated to the 5' terminal sequence end by end, so as to obtain cyclic DNA molecules with different sizes.
(3) A segment of sequence is designed as an upstream primer; the upstream primer is the same as the poly(A) sequence at the 3' terminal of cDNA; the random primer is used to amplify the cyclic DNA molecules, so as to obtain an amplified sequence with poly(T); by means the combination of the poly(A) primer and the poly(T), DNA fragments with different lengths are obtained after amplification; and interruption, library construction and sequencing are performed on these fragments, so as to obtain a full-length RNA sequence.

### See embodiments for details.

In a specific implementation, an overall technology route of full-length RNA sequencing is as follows: droplet (a bead phase including a lysis buffer) preparation→mRNA capture→emulsion breaking→reverse transcription reaction→random primer amplification→cyclization ligation of an amplified product→PCR amplification of a cyclized product→fragmentation for library construction→sequencing. See embodiments for details.

### (II) Strategy of RNA single-cell sequencing at the 5' terminal

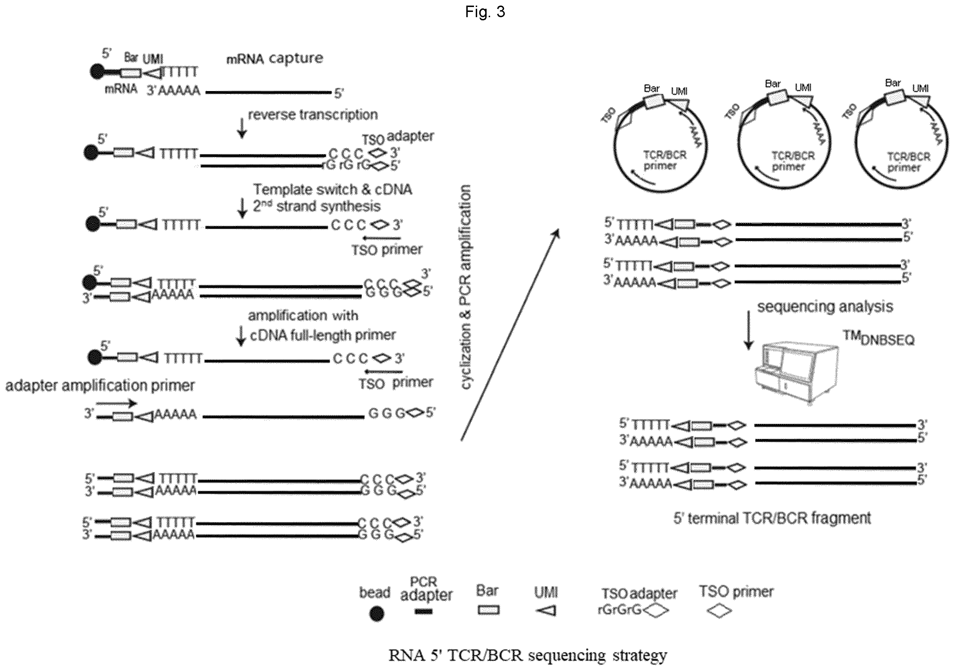

A strategy used for RNA sequencing at the 5' terminal includes: obtaining the mRNA by means of a droplet technology of RNA_seq at the 3' terminal; then performing reverse transcription and amplification on the mRNA, so as to obtain full-length cDNA; performing cyclization ligation on the single-stranded cDNA with the poly(A) in an amplified double-stranded full-length cDNA, so as to make the 3' terminal and the 5' terminal of the mRNA sequence ligated end to end; then using the gene-specific primer (for example, the TCR/BCR primer) complementary to the cyclized cDNA or the random primer and the poly A primer for amplification by further using the ring as the template; and performing fragmentation and screening on these amplified products, and then performing library construction and sequencing, so as to achieve the TCR/BCR sequence capture at the 5' terminal or the 5' sequence capture of other target genes (as shown in Fig. 3).

Specific steps include the following.
(1): A segment of PCR adapter sequence is designed for capturing the poly(A) of the mRNA at the 3' terminal. The PCR adapter sequence may be ligated to the solid support (for example, the bead), and may be used as an adapter amplification primer during cDNA amplification. A segment of TSO primer, which is complementary to the 3' terminal of the first strand cDNA, is designed. After the synthesis of the first strand cDNA, amplification is performed with a primer combination, which is formed by the PCR adapter sequence and the TSO primer, so as to obtain a full-length cDNA fragment.
(2) Through cyclization ligation, cyclization ligation is performed on a single strand with the poly(A) in the full-length cDNA fragment, so as to obtain the cyclic DNA molecules with the cDNA tag sequence at the 3' terminal and the TSO primer at the 5' terminal being ligated to each other end to end.
(3) A segment of sequence is designed as an upstream primer; the primer is the same as the poly(A) sequence at the 3' terminal of cDNA; a segment of TCR/BCR primer or random primer complementary to the cDNA ring is designed; the cyclic DNA molecules are amplified to obtain TCR/BCR fragments at the 5' terminal, or amplified fragments with a random start at the 5' terminal.
(4) Library construction and sequencing are performed on the fragments, which are obtained by means of amplification of the TCR/BCR primer, or the amplified fragments with the random start at the 5' terminal, so as to obtain a TCR/BCR sequence at the 5' terminal of mRNA or a sequence randomly starting from the 5' terminal.

In a specific implementation, an overall technology route of RNA sequencing at the 5' terminal is as follows: droplet (the liquid phase of the bead including a cell lysis buffer) preparation--*emulsion breaking→mRNA capture→reverse transcription reaction--*full-length cDNA amplification→cyclization ligation of an amplified product→PCR amplification on a cyclized product with a ployA primer + a TCR/BCR primer or a random primer→fragmentation for library construction→sequencing.

It can be learned, from the above, that, in the present application, by means of applying the 3' droplet strategy to capture the mRNA, that is, carrying a segment of PCR adapter and cell barcode and UMI sequences on the solid support (for example, the bead), and simultaneously adding a segment of poly(dT) onto the terminal, the poly(dT) is complementary to a poly(A) tail at the 3' terminal of mature mRNA, such that the mRNA is captured, and a first strand cDNA full length is synthesized by means of further reverse transcription. Then cDNA fragments with different sizes or the cDNA full length is obtained by means of amplification with the random primer and ring formation by ligation, such that RNA single-cell sequencing at the 5' terminal and RNA full-length sequencing are achieved.

On the basis of the above improvement ideas and research results, the applicant has proposed the technical solution of the present application. In a typical implementation, the present application provides a method for constructing an RNA sequencing library. The construction method includes the following operations.

A single-stranded cDNA, which is a reverse transcription product of single-cell mRNA, is acquired. The 3'-terminal of the single-stranded cDNA includes a cDNA tag sequence.

The single-stranded cDNA is cyclized to obtain a single-stranded cyclized cDNA.

Amplification is performed with a primer combination, which is formed by a random primer or a gene-specific primer and a cDNA tag primer, so as to obtain an amplified fragment. The cDNA tag primer is at least a part of the cDNA tag sequence.

Fragmentation for library construction is performed on the amplified fragment, so as to obtain the RNA sequencing library.

By means of the above construction method, the single-stranded cDNA, which is a reverse transcription product of mRNA, is obtained, and the 3'-terminal of the single-stranded cDNA includes the cDNA tag sequence; the single-stranded cDNA with the cDNA tag sequence is cyclized to ligate two terminals of the single-stranded cDNA, that is, ligate a 5' terminal and a 3' terminal, which correspond to the mRNA, such that a fragment of the 5' terminal of the mRNA is tagged by means of a 3'-terminal cDNA tag sequence. Then, by means of amplifying single-stranded cyclized cDNA with the cDNA tag primer, which is the same as at least a part of the cDNA tag sequence, and the random primer or the gene-specific primer, an amplified fragment starting from the position of a specific gene at the 5' terminal, or an amplified fragment starting from any position at the 5' terminal; and finally, fragmentation screening is performed on these amplified fragments, and then library construction and sequencing are performed, such that the purpose of high-throughput sequencing is achieved. Therefore, on the basis of whether the length of the obtained single-stranded cDNA used for ring formation is that of a full-length cDNA or a random-length cDNA, a full-length RNA sequencing library, or a 5' RNA sequencing library may be obtained.

It is to be noted that, the construction method is suitable for RNA library construction of any sample, as long as a reversely-transcribed single-stranded cDNA of the mRNA of the sample can be acquired. In particular, the construction method is suitable for single-cell RNA library construction. In a preferred embodiment, the mRNA is derived from a single-cell sample, and is a single-cell mRNA.

The way of acquiring single-cell mRNA may use known methods in the prior art. In a preferred embodiment of the present application, a droplet method is used to prepare the single-cell mRNA, so as to make the single-cell mRNA ligated to a solid support. The solid support is preferably a bead.

In a preferred embodiment of the present application, the operation of using the droplet method to prepare the single-cell mRNA, so as to make the single-cell mRNA ligated to the bead includes: respectively providing a single-cell suspension and the bead, where the bead carries a bead tag sequence, and the terminal of the bead tag sequence includes poly(dT); and wrapping the single-cell suspension and the bead into droplets, where each droplet includes a single cell and one bead, and the bead is combined with the poly(A) of the mRNA in the single-cell suspension by means of the poly(dT), to connect the mRNA in the single-cell suspension to the bead, so as to obtain the single-cell mRNA. It is to be noted that, since the single-cell suspension contains the cell lysis buffer, the single-cell suspension may also be regarded as a cell nucleus suspension. The droplet method achieves the capturing of the mRNA by means of the combination of the poly(dT) on the bead and the poly(A) of the mRNA, such that high capture efficiency is realized. Since a single oil drop corresponds to the single bead and the single cell, the bead tag sequence on the bead can also specifically tag the single cell.

In the above construction method, the specific method for acquiring the single-stranded cDNA, which is a reverse transcription product of mRNA, is not limited, as long as the cDNA tag sequence can be carried at the 3' terminal corresponding to the mRNA. As described above, there is also no particular limitation on the length of the single-stranded cDNA obtained, which may be either full-length cDNA or random-length cDNA, or both. In order to fully exploit and utilize transcriptome information, in a preferred embodiment of the present application, the step of acquiring the single-stranded cDNA, which is the reverse transcription product of mRNA, where the 3'-terminal of the single-stranded cDNA includes the cDNA tag sequence includes: performing reverse transcription on the mRNA, so as to obtain a first strand cDNA; amplifying the first strand cDNA to obtain a double-stranded cDNA, where the 3' terminal of a second strand cDNA, which is complementary to the first strand cDNA, includes the above cDNA tag sequence, and the cDNA tag sequence includes poly(A); and melting the double-stranded cDNA to obtain the single-stranded cDNA including the cDNA tag sequence. The melting of the double-stranded cDNA is performed for subsequent cyclization; and when there is a double strand, it is difficult for the cyclization auxiliary sequence to effectively bind to the strand that needs to be cyclized. The subsequent cyclization method is not limited here, as long as the cDNA can be ligated end to end.

In the above preferred embodiment, the bead tag sequence on the bead for capturing the single cells successively includes, in a direction from 5' to 3', a first PCR adapter, a first cell barcode, a first UMI and the poly(dT); and correspondingly, the cDNA tag sequence successively includes, in a direction from 3' to 5', a second PCR adapter, a second cell barcode, a second UMI and the poly(A). The second PCR adapter is complementary to the first PCR adapter; the second cell barcode is complementary to the first cell barcode; and the second UMI is complementary to the first UMI. That is to say, the 5' terminal of the first strand cDNA carries the bead tag sequence; and the 3' terminal of the second strand cDNA obtained by means of amplification carries the cDNA tag sequence complementary to the bead tag sequence.

In order to facilitate the amplification of single-stranded cDNA, the 5' terminal preferably includes the sequence of the TSO primer. A specific method for carrying the sequence of the TSO primer at the 5' terminal is not limited. In a preferred embodiment of the present application, reverse transcription is performed on the mRNA with a reverse transcriptase having the terminal transferase activity and the TSO adapter, so as to obtain the first strand cDNA. The 3' terminal of the first strand cDNA includes a complementary sequence of the TSO adapter. The first strand cDNA is amplified to obtain the second strand cDNA, and the 5' terminal of the second single-stranded cDNA includes the sequence of the TSO primer.

Specifically, when the first strand cDNA is amplified, the full-length second strand cDNA may be obtained if the TSO primer is used; and if the TSO-random primer is used for amplification, the second strand cDNA with any length may be obtained. In this way, according to different types of the amplified primers, the double-stranded cDNA with a full length or any length may be obtained, such that the full-length or any length single-stranded cDNA with the cDNA tag sequence may be obtained after melting.

The sequence of the TSO adapter may use an existing known sequence or may be automatically designed according to requirements. In some preferred embodiments of the present application, the sequence of the TSO adapter is SEQ ID NO: 1. The reverse transcriptase includes, but is not limited to, an Alpha reverse transcriptase of MGI, a Superscript^{™}II reverse transcriptase of Invitrogen, Superscript IV of Thermo, or Maxima H Minus of Thermo.

It is to be noted that, a method for capturing the mRNA of single cells on the basis of a droplet method is the method that truly achieves low-cost and high-throughput transcriptome sequencing at present. The core of the method is to use a droplet as a micro-reactor; and the droplet includes a cell and a carrier/support including a tag sequence (generally, a first cell barcode sequence and a UMI have been included in advance). The carrier/support is preferably a bead. After the droplet is formed, the mRNA is released after cell lysis, and is combined with a capture sequence on the bead, so as to achieve the capture of the mRNA. Generally, after the mRNA is enriched in the droplets, the mRNA (in this case, the mRNA has carried a tag complementary to the bead tag sequence, and thousands of cells may be treated at the same batch) enriched by all the beads are merged for subsequent library construction. The construction of an mRNA library of 10× Genomics uses the above principle, but the disadvantages thereof are that the 10×Genomics mainly aims at library construction and sequencing at the 3' terminal of the mRNA, but is difficult to achieve library construction and sequencing for the full length of the mRNA.

In the above preferred embodiment of the present application, the step of capturing the mRNA of single cells by means of the droplet method is the same as that of the above method. The process of obtaining the first strand cDNA by performing reverse transcription on the mRNA is also the same as that of the current method; and the reverse transcriptase having the terminal transferase activity may be used to perform reverse transcription, so as to obtain the first strand full-length cDNA. Due to the terminal transferase activity of the reverse transcriptase, three Cs can be added to the terminal of the first strand full-length cDNA. In this case, three rGrG+G at the terminal of the free TSO adapter (see SEQ ID NO: 1 in Embodiment 1 for examples) in the droplet can be combined with the three Cs; and next, a sequence (see the sequence of the underlined part of SEQ ID NO: 1 in Embodiment 1 for examples) complementary to the TSO adapter is synthesized at the downstream of the CCC at the terminal of the first strand full-length cDNA. Then the synthesis of the second strand cDNA is completed with the TSO primer or the TSO-random primer. In this way, the obtained double-stranded cDNA is either the full-length cDNA or the cDNA with a fragment of any length; and the 3' terminal corresponding to the mRNA includes the cDNA tag sequence. As described above, in the present application, the cDNA tag sequence may be transferred by cyclizing one strand (that is, the single-stranded cDNA with the poly(A)) in such double-stranded cDNA, such that a non-full-length amplified fragment tagging the 5' terminal of the mRNA, or even a full-length amplified fragment, is obtained; and 5' terminal and/or full-length sequencing of the mRNA may be achieved by constructing these amplified fragments into a library.

Therefore, random amplification or full-length amplification may be performed on the first strand cDNA according to actual requirements. In order to further increase the comprehensive coverage of the above amplified fragment to the 5' terminal fragment of mRNA, even covering the full length, in a preferred embodiment of the present application, random amplification or full-length amplification is performed on the first strand cDNA, so as to obtain the double-stranded cDNA. In another preferred embodiment, with the adapter amplification primer (see SEQ ID NO:2 in Embodiment 1 for examples), and at least one of the TSO primer (see SEQ ID NO:3 in Embodiment 1 for examples) or the TSO-random primer (see SEQ ID NO:4 in Embodiment 1 for examples), the first strand cDNA is amplified to obtain the double-stranded cDNA.

By means of the TSO-random primer, a double-stranded cDNA fragment starting from any position at the 5' terminal may be obtained by means of amplification, such that fragments of all sequences of the mRNA from the 5' terminal to the 3' terminal are covered as comprehensively as possible. The TSO primer can ensure that a full-length double-stranded cDNA fragment is obtained. By means of cyclizing the fragments with different lengths, single-stranded cyclic DNA molecules with different sizes may be obtained, and all of cyclic fragments are amplified, so as to obtain amplified fragments at different positions that can tag the 5' terminal of the mRNA; and library construction is performed on these amplified fragments, so as to obtain a sequencing library covering fragments at the 5' terminal and/or full length of the mRNA at different positions.

The step of cyclizing the single-stranded cDNA may use an existing cyclization method. For example, a cyclization means in an MGI cyclization sequencing technology is used for implementation. In a preferred embodiment of the present application, the operation of cyclizing the single-stranded cDNA to obtain the single-stranded cyclized cDNA includes: ligating the single-stranded cDNA into a ring under the action of a cyclization auxiliary sequence and a ligase, so as to obtain a ligated product; and performing enzyme digestion on the ligated product to digest the single-stranded cDNA, which is not ligated into the ring (if the double-stranded DNA is melted and directly cyclized without separation, there may also be uncyclized double-stranded DNA here), so as to obtain the single-stranded cyclized cDNA. The cyclization auxiliary sequence (see SEQ ID NO:5 in Embodiment 1 for examples) is complementary to sequences on two terminals of the cyclized single-stranded cDNA (for example, being complementary to the second PCR adapter in the cDNA tag sequence, and complementary to the TSO primer).

In a more preferred embodiment, heat denaturation is first performed on the double-stranded cDNA to melt the double-stranded cDNA into two single strands; the cyclization auxiliary sequence (reasonably designed according to the sequences at the two terminals of the single strand that needs to be cyclized) is incubated with the single strand in a single-stranded state; and the two terminals of the cyclization auxiliary sequence are closer to each other by being complementary to the sequences at the two terminals of the single strand, such that single-stranded cyclization is achieved under the action of the ligase.

After the single-stranded cyclized cDNA is obtained, amplification may be performed with the poly(A) primer and the random primer or the gene-specific primer of the specific type (for example, the TCR/BCR primer) according to the actual purpose of the study, so as to obtain different amplified fragments tagging the 5' terminal. By means of the method, the existing method for tagging the 3' terminal of the mRNA is converted to a method for tagging a target fragment at the 5' terminal of the mRNA by means of cyclization first and then amplification, thus achieving 5' terminal sequencing and/or full-length sequencing of the mRNA. The method is simple and convenient, and is compatible with library construction steps of various existing sequencing platforms, thereby facilitating the high-throughput sequencing at the 5' terminal and/or full length of the mRNA of the single cells.

In a preferred embodiment, the single-stranded cyclized cDNA is amplified with a combination of at least one of the following primers and the cDNA tag primer, so as to obtain amplified fragments meeting different requirements: (a) a random primer; (b) a TCR gene primer; or (c) a BCR gene primer. Preferably, the cDNA tag primer is the poly(A) primer, more preferably SEQ ID NO: 6. As described above, in case of studying an immunome library, primers for TCR and/or BCR genes are used, thereby obtaining the expression of genes related to immunity.

The step of performing fragmentation for library construction on the amplified fragment, so as to obtain the RNA sequencing library of the single cell uses a conventional process of fragmentation for library construction. In a preferred embodiment of the present application, the step includes: adding a library adapter to the amplified fragment, so as to obtain the RNA sequencing library. The specific way of adding the adapter may select an appropriate library adapter and an operation mode according to different sequencing platforms. In a preferred embodiment of the present application, the step includes: performing fragmentation with enzyme digestion on the amplified fragment, so as to obtain digested fragments; and successively performing terminal repair, A tailing and library adapter ligation on the digested fragments, so as to obtain the RNA sequencing library. More preferably, after terminal repair, A tailing and adapter ligation are performed, the method further includes amplifying the obtained ligated fragments, so as to obtain the RNA sequencing library required for computer operating.

In the step of adapter ligation, according to different sequencing platforms, adapters suitable for a particular sequencing platform may be reasonably selected. For example, the adapter may be either an adapter of an MGI sequencing platform or an adapter of an Illumina sequencing platform. Correspondingly, the amplified primer used to amplify the ligated fragments after the adapter is ligated is also paired with the corresponding platform adapter sequences. For example, if the adapter of the MGI sequencing platform is used, the primer used to amplify the ligated fragments is also the amplified primer of the MGI sequencing platform.

A second typical implementation of the present application further provides a single-cell RNA library construction kit. The kit includes: a cyclization auxiliary sequence, a DNA ligase, a cDNA tag primer, and at least one of the following primer sequences: (a) a random primer; (b) a TCR gene primer; or (c) a BCR gene primer.

The kit is mainly designed on the basis reagents used in the above cyclization step and the step of amplifying the specific fragment at the 5' terminal of the target gene on the single-stranded cyclized DNA or the random fragment starting from any position at the 5' terminal in the above library construction method; and a library is conveniently and rapidly constructed by including the reagents. The cyclization auxiliary sequence is separately complementary to and combined with the TSO adapter added corresponding to the 5' terminal of the mRNA and the tag sequence at the 3' terminal of the mRNA, such that the specific sequence composition also varies according to the tag sequence and the specific sequence on the TSO adapter.

The DNA ligase in the kit is mainly used to ligate a phosphorylation-modified base and a base with a hydroxyl group in a DNA strand, such that any DNA ligase that can implement DNA ligation is applicable to the present application. Specifically, it may be a thermally unstable DNA ligase, such as a T4 DNA ligase, or may be a thermally stable DNA ligase, such as a Thermo stable DNA ligase.

For ease of construction of the library, in a preferred embodiment, the kit further includes a solid support. The solid support is provided with a support tag sequence. The cDNA tag primer is complementary to at least a part of the support tag sequence. Preferably, the solid support is a bead, and the support tag sequence is a bead tag sequence. The solid support with the support tag sequence can facilitate the capture of single-cell mRNA while allowing the mRNA to carry the tag sequence complementary to the support tag sequence.

In the kit, the bead with the tag sequence (for example, gel bead) may be selectively purchased from existing beads or may be self-prepared. The tag sequence on each bead includes the following DNA sequences: (1) a PCR adapter, which is used for PCR amplification; (2) a cell barcode, one bead corresponding to one cell barcode; (3) a UMI, which is used for tagging different template molecules in the same cell, and for quantifying the abundance of transcripts; and (4) a capture sequence, which is generally poly(dT), and is used for capturing the mRNA by combining the poly(A) tail of the mRNA.

In order to further improve the convenience of library construction, the kit preferably further includes an RNA extraction reagent and/or an RNA reverse transcription reagent. The RNA reverse transcription reagent includes the reverse transcriptase. The reverse transcriptase is the reverse transcriptase having the terminal transferase activity (for example, which may be an Alpha reverse transcriptase of MGI, a Superscript^{™}II transcriptase of Invitrogen, or may be Maxima H Minus of Thermo, or Superscript IV).

Related reagents for mRNA capture and reverse transcription based on the droplet method may be used together. After poly(dT) captures the mRNA by combining with the poly(A) tail of the mRNA, the synthesis of the first strand cDNA is achieved under the action of the reverse transcriptase. For ease of synthesis of a second strand, in addition to including the reverse transcriptase, the usual reverse transcription reagent further includes the TSO adapter (for example, as shown SEQ ID NO: 1). For example, by means of using the reverse transcriptase having the terminal transferase activity, CCC can be added to the terminal of the first strand cDNA; then after the rGrG+G on the TSO adapter is used to be complementarily combined with the CCC, with a TSO adapter sequence as a template, the complementary sequence (that is, equivalent to ligating the complementary sequence of TSO to the terminal of the first strand cDNA) of the TSO adapter is added after the CCC.

In order to obtain a sequence fragment of the mRNA at any position of the 5' terminal to the 3' terminal, to obtain all fragments covering the full length of the mRNA by means of a plurality of fragments with different lengths, so as to achieve sequencing on the 5' terminal or sequencing on the full length, in a preferred embodiment of the present application, the kit further includes a TSO primer, a TSO-random primer and an adapter amplification primer. Preferably, the sequence of the adapter amplification primer is SEQ ID NO: 2; the sequence of the TSO primer is SEQ ID NO: 3; and the sequence of the TSO-random primer is SEQ ID NO: 4. The adapter amplification primer can be combined with the cDNA tag sequence at the 3' terminal of the second strand cDNA (corresponding to the 3' terminal of the mRNA); and the TSO-random primer can be combined with any position at the 3' terminal of the first strand cDNA (corresponding to the 5' terminal of the mRNA), such that cDNA fragments with different lengths can be obtained.

Preferably, the cyclization auxiliary sequence is SEQ ID NO: 5; and preferably, the cDNA tag primer is the poly(A) primer, preferably SEQ ID NO: 6.

In some preferred embodiments, the kit further includes at least one of an exonuclease or a library adapter. The exonuclease is used for degrading uncyclized single-stranded or double-stranded cDNA after the double-stranded cDNA is melted into a single strand for cyclization. For example, the exonuclease may be exonuclease I or exonuclease III. The adapter used for library construction may be the adapter of the MGI sequencing platform (for example, one is a linear adapter, and one is a double-stranded adapter of a bubble adapter, wherein the linear adapter is A+31bp sequence + 10bp index sequence + 17bp, the bubble adapter includes a bubble sequence of 17bp, 13bp before the bubble sequence of 17bp and 7bp+T after the bubble sequence of 17bp, and the total length of the adapter is 97bp), or may be adapters of other sequencing platforms, for example, the adapter of the Illumina sequencing platform (for example, Y-type P5 and P7 adapters, and one or two of the P5 and P7 adapters carry a library tag sequence according to requirements, so as to facilitate subsequent splitting of output data of the library of mixed sample sequencing).

A third typical implementation of the present application further provides an RNA sequencing method. The method includes: using any one of the foregoing RNA sequencing library construction methods to construct an RNA sequencing library, and performing sequencing on the RNA sequencing library. The RNA sequencing library, which is constructed with the above RNA sequencing library construction method, according to different research purposes, may be a fragment covering more 5' terminals of the mRNA, or may be a fragment covering the full length of the mRNA, such that current market requirements for 5' terminal sequencing can be met, for example, requirements for 5' terminal sequencing during the construction of an immunopeptide library. Sequencing of the full-length RNA sequencing library can meet the study of structural variation in variable splicing of certain transcripts.

It is to be noted that, the above 5' end RNA sequencing and full-length sequencing may be performed simultaneously or separately, depending on a specific application scenario.

The technical effects of the present application are further described below with reference to specific embodiments.

The following embodiments include cell suspension preparation, bead preparation, droplet generation, emulsion breaking, reverse transcription RT reaction, cDNA amplification, cyclization ligation, cyclized product amplification, fragmentation enzyme library construction, high-throughput sequencing, and the like.

### Embodiment 1 full-length RNA sequencing

In this embodiment, preparation is performed according to a principal process shown in Fig. 2, and specifically includes the following.

### 1. Single-cell suspension preparation

1.1 For a cell line and solid tissue, an appropriate digestion method/grinding method was used to prepare a single cell/cell nucleus suspension; washing was performed with PBS (containing 0.04%BSA) for 1-2 times; and filtration was performed with a 40µm cell sieve.

1.2 A cell counting chamber or a counter to measure the concentration of cells or cell nuclei.

1.3 According to the concentration of cells, 100,000 cells/cell nuclei were extracted; centrifugation was performed at 300-500 g at 4 °C for 5 min, and then cell precipitation was collected; 100µL of a cell resuspension buffer (0.04%BSA+PBS) was added to resuspend the cells or cell nuclei.

### 2 Bead preparation

2.1 200µL (220,000) magnetic beads were sucked to a 0.2mL PCR tube, and then was placed on a magnetic separator to stand for 2 min; and supernatant was discarded.

2.2 The PCR tube was removed from the magnetic separator; 200µL of 1x Buffer D (1mM EDTA, 9mg/mL 85% KOH) was added to suspend the magnetic beads; and incubation was performed for 5 min at room temperature.

2.3 Standing was performed for 2 min by placing the tube on the magnetic separator; and supernatant was removed.

2.4 The PCR tube was held on the magnetic separator; 200µL of 1x Buffer D was added; and standing was performed for 30s, and then supernatant was removed.

2.5 200µL of LSWB (50mM TrTSO-HCl, 150mM NaCl, 0.05%Tween-20) was added; standing was performed for 30s; then supernatant was removed; and the previous operation was repeated.

2.6 200µL of a lysis buffer (6%Ficoll PM-400, 0.2%Salbutamol, 20mM EDTA, 200mM Tris pH 7.5, H₂O) was added; standing was performed for 30s; then supernatant was removed; the PCR tube was removed from the magnetic separator; and 100µL of the lysis buffer and 5µL of 1M DTT were added.

### 3 Droplet generation

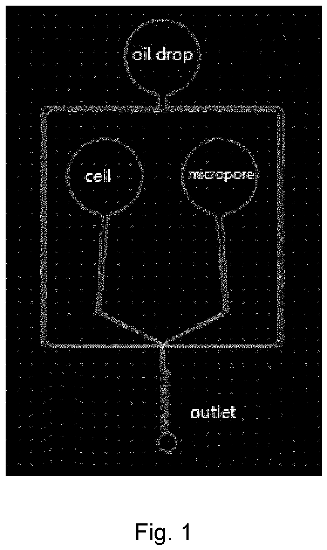

3.1 A surface protective film of a chip (as shown in Fig. 1) was torn off; and then the chip was placed in a chip slot region of a droplet generation apparatus (10× Genomics).

3.2 End A of a connection tube (the connection tube being in contact with the bottom of a collection tube) on a collection cover was inserted into an outlet of the chip.

3.3 A 50 mL syringe was placed on a fixation frame, and a push rod was adjusted to an initial position; and a plain end needle was connected to the syringe and an End B of a connection tube (the connection tube being not in contact with the bottom of the collection tube) on the cover of the collection tube.

3.4 200µL of droplet formation oil was added to the collection tube; then the collection cover was tightly screwed; and the collection tube was vertically placed on the fixation frame.

3.5 The cells are well mixed by gently blowing with a pipette; and 100 µL of the cell suspension prepared in step 1.3 was added to the cell wells of the chip, making sure that a pipette tip was in contact with the bottoms of the wells.

3.6 The magnetic beads are well mixed by gently blowing with a pipette; and 100 µL of the magnetic beads was added to bead wells, making sure that the pipette tip was in contact with the bottoms of the wells.

3.7 350µL of the droplet formation oil was immediately added to oil wells of the chip.

3.8 The push rod of the syringe was quickly pulled to the position of a clamping groove, and the push rod was clamped in the clamping groove.

3.9 A timer was started for 20 min, and droplets were collected.

3.10 After 20 min, the collection cover on the collection tube was immediately unscrewed, the connection tube at the outlet of the chip was pulled and vertically stretched, and the droplets in the tube flow into the collection tube; and then a common cover of the collection tube was replaced.

3.11 Standing was performed for 20 min at room temperature, so as to make mRNA molecules fully combined with the magnetic beads.

### 4 Emulsion breaking

4.1 An emulsion breaking reagent was prepared; and 10mL of 6X SSC (20X SSC, Invitrogen, diluted to 6x with enzyme-free water) and 200µL of Perfluorooctanol (PFO, Sigma, 370533-25G) are added into a 15 mL centrifuge tube.

4.2 A filtering apparatus was connected to a vacuum pump; a pressure parameter was adjusted to 0.01 MPa or 100 mbar; and the vacuum pump was started.

4.3 20mL of 6X SSC was added, and pre-processing was performed on the apparatus.

4.4 When there was no liquid remaining on a filter membrane, all liquid in the collection tube was uniformly poured on the surface of the filter membrane; the collection tube was washed twice with 2mL of 6X SSC; and then the cleaning liquid was poured into the filtering apparatus together.

4.5 10 mL of the emulsion breaking reagent was well mixed by means of vigorous inversion, and then was slowly poured into the filtering apparatus in batches.

4.6 When there was no liquid remaining on the filter membrane, 30mL of 6X SSC was continuously added, and the magnetic beads were washed in batches.

4.7 When there was no liquid remaining on the filter membrane, the vacuum pump was closed, and the vacuum pump and the filtering apparatus were disconnected.

4.8 A filtering port of the filtering apparatus was sealed with the syringe or a rubber stopper.

4.9 1.0 mL of the collected buffer was added with a pipette, the surface of the entire filter membrane was gently blown for about 20 times, and then the magnetic beads were suspended.

4.10 The collected buffer containing the magnetic beads was transferred into a 1.5 mL low-absorption centrifuge tube.

4.11 1.0 mL of the collected buffer was used, the surface of the entire filter membrane was gently blown for about 10 times, and then the residual magnetic beads were suspended.

4.12 The collected buffer containing the magnetic beads was transferred into the 1.5 mL low-absorption centrifuge tube, the centrifuge tube was placed on the magnetic separator and allowed to stand for 2 min, and then supernatant was slowly removed.

4.13 The centrifuge tube was removed from the magnetic separator, 100µL of collected buffer was used to successively suspend and adsorb the magnetic beads on sides of the two centrifuge tubes, and liquid was transferred into a 0.2mL low-adsorption PCR tube.

4.14 100µL of the collected buffer was used again to successively suspend and adsorb the magnetic beads on sides of the two centrifuge tubes, and liquid was transferred into the low-adsorption PCR tube.

4.15 The PCR tube containing the magnetic beads on the magnetic separator and was allowed to stand for 2 min; and then supernatant was removed.

4.16 A magnetic bead adsorption state was maintained, 200µL of 6X SSC is added, standing was performed for 30s, and then supernatant was removed.

4.17 200µL of 5X FS Buffer (MGI, 01 E022MS) was added, standing was performed for 30s, and supernatant was slowly removed, so as to prevent the magnetic beads from being adsorbed.

### 5 Reverse transcription reaction

5.1 Preparation of a reverse transcription reaction system on ice: 5µL of H₂O, 20µL of 5x First-Strand Buffer (FS Buffer) , 20µL of 5M Betaine, 10µL of 10mM dNTPs, 7.5µL of 100mM MgCl₂, 5µL of 50µM Template switch oligo (TSO adapter), 5µL of 100mM DTT, 5µL of 200U/µL SuperScriptTMII reverse transcriptase (Invitrogen, 18064014), 2.5µL of 40U/µL RNase inhibitor.

TSO adapter sequence: SEQ ID NO : 1 : 5'-AAGCAGTGGTATCAACGCAGAGTACATrGrG+G-3', wherein +G represents locked nucleic acid; and the reason for using rGrG+G was the better thermal stability of the hybridization of RNA with DNA.

5.2 100µL of the reverse transcription reaction system was extracted and added to the PCR tube holding the magnetic beads in step 4.17; and then blowing was performed for well mixing.

5.3 A reverse transcription reaction was performed according to the following conditions: 42 °C, 90 min, 10 cycles (50 °C, 2 min; 42 °C, 2 min), and a hot lid temperature being set to 75 °C. Due to the settlement phenomenon of the magnetic beads, the beads were flicked and well mixed at 20 min intervals, and after the beads were centrifuged briefly, the reaction was continued.

5.4 After the reaction was finished, brief centrifugation is performed, the PCR tube was placed on the magnetic separator to stand for 2 min, and a reaction solution was removed.

5.5 The PCR tube was removed from the magnetic separator, 200µL of TE-SDS(TE Buffer+0.5%SDS) was added and shaken for well mixing, and then the reaction was terminated.

5.6 Brief centrifugation was performed, the PCR tube was placed on the magnetic separator to stand for 2 min, and liquid was removed.

5.7 The magnetic bead adsorption state was maintained, 200µL of TE-TW (TE Buffer+0.01 % Tween-20) was added, standing was performed for 30s, and then supernatant was removed.

5.8 The above step was repeated.

5.9 Magnetic bead adsorption was maintained, 200µL of 10mM NF-H₂O was added, standing was performed for 30s, and then the supernatant was removed.

### 6. Random primer amplification of a first strand cDNA

6.1 Preparation of a PCR system: 42µL of H₂O, 4µL of a 10µM Tn primer (that is, an adapter amplification primer), 2µL of a 20µm TSO-random primer, 2µL of a 20µm TSO primer and 50µL of 2x KAPA HiFi Hotstart Ready mix (KAPA: KK2602).

The Tn primer (that is, the adapter amplification primer) was used to amplify from one terminal ligated to the magnetic beads, and a specific sequence thereof was:
SEQ ID NO: 2: 5'-CGTAGCCATGTCGTTCTG-3'.

The TSO primer was used to amplify from one terminal of the TSO adapter, and a specific sequence thereof was:
SEQ ID NO: 3: 5'Phos-AAGCAGTGGTATCAACGCAGAGTACAT-3'.

The TSO-random primer was used to amplify from any position at the 5' terminal of the cDNA to the 3' terminal, and a specific sequence thereof was:
SEQ ID NO: 4: 5'phos-AAGCAGTGGTATCAACGCAGAGTACATNNNNNN-3'.

6.2 The PCR was performed according to the following conditions: 95 °C, 3 min; 10-15 cycles (98°C, 20s; 58°C, 20s; 72°C, 3 min); 72°C, 5 min; 4°C, keeping the temperature.

6.3 After the PCR was finished, 120µL of (1.2x) VAHTSTM DNA Clean Beads (VAZYME: N411-03) (balancing being performed for 30 min at room temperature in advance) was used to purify and recycle a PCR product.

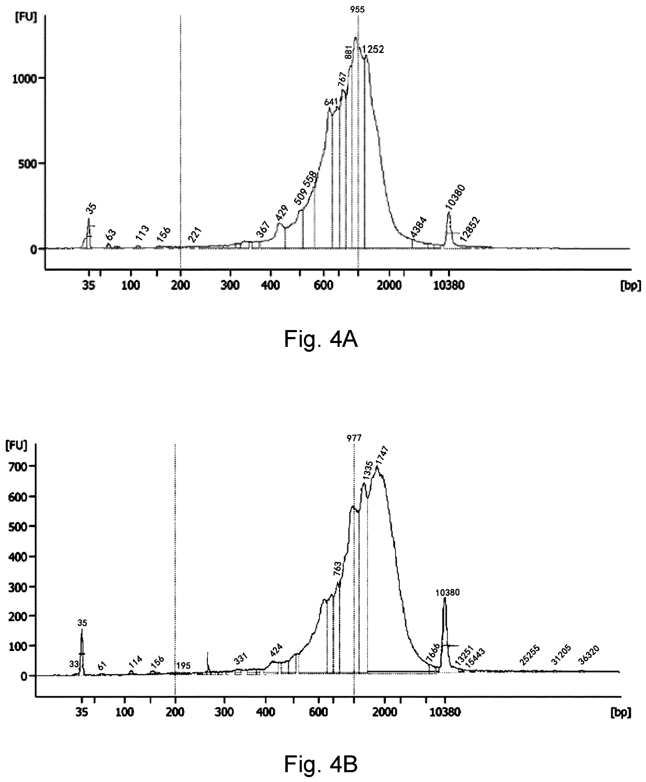

6.4 The PCR purified product was quantified with a Qubit fluorimeter, and an Agilent 2100 biological analyzer was used for detecting the distribution of fragments (see Figs. 4A and 4B for a cell line sample and a solid tissue sample, respectively).

### 7. DNA cyclization

7.1 100-200 ng of a DNA product was taken, the volume was made up to 45µL with NF-H₂O, 5µL of splint oligo 1 (20µm) was added, short-term vortex was performed for well mixing, instantaneous centrifugation was performed for 5s, a reaction was performed at 95 °C for 3 min (a hot lid being 105°C, so as to melt the dsDNA into a single strand, thereby facilitating single strand cyclization), and the mixture was placed on ice rapidly for 5-10 min.

A specific sequence of the splint oligo 1 (cyclization auxiliary sequence) is:
SEQ ID NO: 5: 5'-TACCACTGCTT CGTAGCCATGT-3'.

### 7.2 Ligation

The PCR tube was placed into an ice bath, and the reaction system was prepared according to the table below.

**Table 1**

| Component | Volume |
|---|---|
| Ligation Buffer B | 9.8µL |
| T4 DNA ligase | 0.2µL |
| Total | 10.0µL |

The well-prepared ligated product was added into a melted product, short-term vortex was performed for well mixing, brief centrifugation is performed, the PCR tube was placed on a PCR instrument, incubation was performed for 45 min at 37 °C, and the hot lid temperature was 75°C.

### 7.3 Enzymatic digestion

When a single strand cyclization reaction was nearly finished, an enzymatic digestion reaction solution was prepared on ice according to the table below.

**Table 2**

| Component | Volume |
|---|---|
| Exonuclease B | 2.6µL |
| Exonuclease Buffer B | 1.4µL |
| Total | 4.0µL |

4µL of the well-prepared enzymatic digestion reaction solution (which was used for digesting uncyclized single chains and possibly unmelted double strands) was pipetted with a pipette and added into a single-stranded cyclized product, short-term vortex was performed for well mixing, instantaneous centrifugation was performed, the PCR tube was placed on a PCR instrument, incubation was performed for 30min at 37 °C, and the hot lid temperature was 75 °C.

7.4 Enzyme digestion termination: after an enzyme digestion reaction was finished, 3µL of a stop solution (0.1M EDTA) was added into the PCR tube, well mixing was performed, and brief centrifugation was performed to collect liquid to the bottom of the tube.

7.5 A cDNA cyclization library was purified, PEG32 magnetic beads were used to purify the cyclized product obtained in step 7.4, the Qubit fluorimeter was used to quantify the purified cDNA cyclized product for use of subsequent amplification.

### 8 PCR amplification of a cDNA random primer

8.1 The cyclized DNA product starting at 10-50 ng is taken, the volume was made up to 42µL with NF-H₂O; and 4µL of a 20µm poly(A) primer, 4µL of the random primer (SEQ ID NO: 12: 5'-NNNNNN-3') and 50µL of 2x KAPA HiFi Hotstart Ready mix were added. Such primers were able to perform amplification of fragments with different lengths on the cyclized products with different sizes, thus the full-length sequence of the cDNA was covered as much as possible.

A specific sequence of the poly(A) primer was:
SEQ ID NO: 6: 5'phos-AAAAAAAAAAAAAAAAAAAAAAAAAAAAAA-3'.

8.2 The PCR was performed according to the following conditions: 95 °C, 3 min; 10-15 cycles (98 °C, 20s; 60 °C, 20s; 72 °C, 30s); 72 °C, 5 min; 4 °C, keeping the temperature.

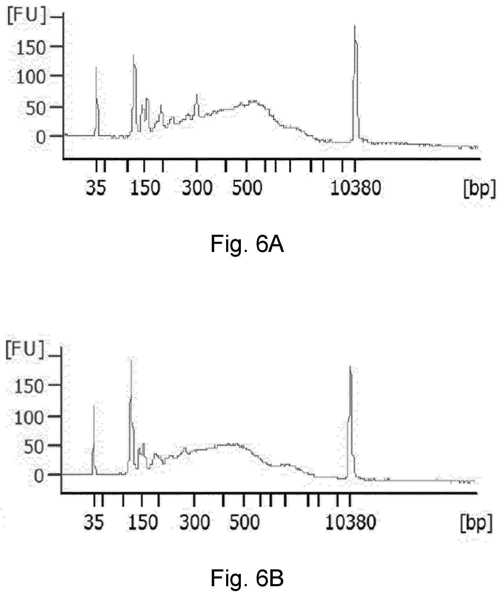

8.3 0.5×+0.7× VAHTSTM DNA Clean Beads magnetic beads were used for PCR product purification, Qubit quantification was performed on the screened fragments (a length being 150-800bp), and the Agilent 2100 biological analyzer was used for detecting the distribution of fragments (see Figs. 6A and 6B for a cell line sample and a solid tissue sample, respectively).

### 9 cDNA library construction

### 9.1 DNA fragmentation

According to a cDNA concentration obtained in step 8.3, 100-200 ng (about 0.1-0.2 pmol) of cDNA to be interrupted was taken into a new 0.2mL PCR tube, the volume should be less than or equal to 16µL, and the part less than 16µL was made up with H₂O. A fragmentation reaction solution was prepared on ice according to the table below.

**Table 3**

| Component | Volume | System |
|---|---|---|
| Fragmentation Buffer | 2.0µL | 1X |
| Fragmentation Enzyme | 2.0µL | / |
| Total | 4.0µL | / |

The PCR tube was placed on the PCR instrument; the hot lid was set to 75°C; incubation was performed for 10 min at 37 °C; after the reaction was finished, 30µL of 0.1M EDTA was added into the PCR tube, vortex shaking was performed for well mixing; and then the reaction was terminated.

9.2 Purification of a fragmented product: the interrupted DNA product was purified and screened (reserving 300-500bp of fragments) with 0.6×+0.2× VAHTSTM DNA Clean Beads magnetic beads, and the Qubit fluorimeter was used to quantify the concentration.

9.3 A terminal repair reaction solution was prepared on ice according to the table below.

**Table 4**

| Component | Volume |
|---|---|
| ER&A-tailing Buffering | 8.5µL |
| ER&A-tailing Enzyme | 1.5µL |
| Total | 10µL |

10 µL of the well-prepared terminal repair reaction solution was pipetted with the pipette and was added into the fragmented product purified in step 9.2, short-term vortex was performed for well mixing, instantaneous centrifugation was performed, the PCR tube was placed on the PCR instrument, 37 °C, 30 min, 65 °C, 15 min, 4 °C, keeping the temperature.

### 9.4 Library adapter ligation

A adapter ligation reaction solution was prepared on ice according to the table below.

**Table 5**

| Component | Volume |
|---|---|
| Ligation Buffer A | 23.4µL |
| 10µM adapter | 5.0µL |
| DNA ligase | 1.6µL |
| Total | 30µL |

A specific sequence of the library adapter was:
SEQ ID NO: 7: 5'-Phos-AGTCGGAGGCCAAGCGGTCTTAGGAAGACAA-3'; and
SEQ ID NO: 8: 3'-TTCAGCCTCCGGT-5'.

30 µL of the well-prepared adapter ligation reaction solution was slowly pipetted with the pipette and was added into a terminal repair product purified, vortex shaking was performed for well mixing, instantaneous centrifugation was performed, and the reaction solution was collected at the bottom of the tube, the PCR tube was placed on the PCR instrument, 23°C, 30 min, 4°C, keeping the temperature.

9.5 Purification of a ligated product: 1.0× VAHTSTM DNA Clean Beads magnetic beads were used for purification, and the Qubit fluorimeter was used to measure the concentration of the ligated product.

### 9.6 PCR amplification of an adapter ligation product

A specific sequence of the primer amplifying the adapter ligation product was:
FP:5'-phos-AAAAAAAAAAAAAAAAAAAAAAAAAAAAAA-3' (SEQ ID NO: 9); and
RP:5'-TGTGAGCCAAGGAGTTGNNNNNNNNNNTTGTCTTCCTAAGACCGCT-3' (SEQ ID NO: 10),
wherein NNNNNNNNNN was a tag sequence; and N represents any one of A/T/C/G, and was used for distinguishing different libraries. A PCR reaction mixed solution was prepared in a centrifuge tube according to the table below.

**Table 6**

| Component | Volume |
|---|---|
| 2X KAPA HiFi Hotstart Ready mix | 50µL |
| FP (10µM) | 2µL |
| RP (10µM) | 2µL |
| Ligated purified product | 46µL |
| Total | 100µL |

54 µL of the well-prepared PCR reaction mixed solution was pipetted with the pipette and added into the purified ligated product; vortex shaking was performed for well mixing, instantaneous centrifugation was performed, and the reaction solution was collected at the bottom of the tube for PCR amplification: 95°C, 3 min; 10-15cycles (98°C, 20s; 60°C, 20s; 72°C, 30s); 72°C, 5min; 4°C, keeping the temperature.

9.7 Fragment screening of PCR amplification products: (0.6×+0.6×)VAHTSTM DNA Clean Beads magnetic beads were used for purification, and the Qubit was used for product quantification.

### 10 High-throughput sequencing

10.1 Denaturation: 200-400ng of the DNA product is taken, the volume was made up to 47µL with NF-H₂O, 3 µL of splint oligo 2 (20µm) was added, short-term vortex was performed for well mixing, instantaneous centrifugation was performed for 5s, a reaction was performed at 95 °C for 3 min (a hot lid being 105 °C), and the mixture was placed on ice rapidly for 5-10 min.

(splint oligo 2:5'-TTTTTTTTTTTTGTGAGCCAAG-3') (SEQ ID NO: 11, and the sequence of the underlined part being the same as the first 11 positions of a FP sequence in SEQ ID NO: 10).

### 10.2 Ligation

The PCR tube was placed into an ice bath, and the reaction system was prepared according to the table below.

**Table 7**

| Component | Volume |
|---|---|
| Ligation Buffer B | 9.8µL |
| DNA ligase | 0.2µL |
| Total | 10µL |

The well-prepared ligated product was added into a melted product, short-term vortex was performed for well mixing, brief centrifugation was performed, the PCR tube was placed on a PCR instrument, incubation was performed for 30min at 37 °C, and the hot lid temperature was 75 °C.

### 10.3 Enzymatic digestion

When a single strand cyclization reaction was nearly finished, an enzymatic digestion reaction solution was prepared on ice according to the table below.

**Table 8**

| Component | Volume |
|---|---|
| Exonuclease B | 2.6µL |
| Exonuclease Buffer B | 1.4µL |
| Total | 4.0µL |

4 µL of the well-prepared enzymatic digestion reaction solution was pipetted with a pipette and added into a single-stranded cyclized product, short-term vortex was performed for well mixing, instantaneous centrifugation was performed, the PCR tube was placed on a PCR instrument, incubation was performed for 30min at 37°C, and the hot lid temperature was 75 °C.

10.4 Enzyme digestion termination: after an enzyme digestion reaction was finished, 3µL of a stop solution (0.1M EDTA) was added into the PCR tube, vortex was performed for well mixing, and brief centrifugation was performed to collect liquid to the bottom of the tube.

10.5 Purification of a cyclized library: PEG32 magnetic beads were used to purify the cyclized product, the Qubit fluorimeter was used to quantify the purified product, it was required that the quality of the library > 0.5 ng/µL, the library was qualified, an MGISEQ2000 high-throughput sequencer was used for sequencing.

### Embodiment 2 RNA single-cell sequencing at the 5' terminal

In this embodiment, preparation was performed according to a principal process shown in Fig. 2, and specifically includes the following.

### (I) Single-cell mRNA capture and synthesis of a first strand cDNA

The steps were the same as the steps of full-length RNA sequencing.

### (II) The step of cDNA amplification includes the following.

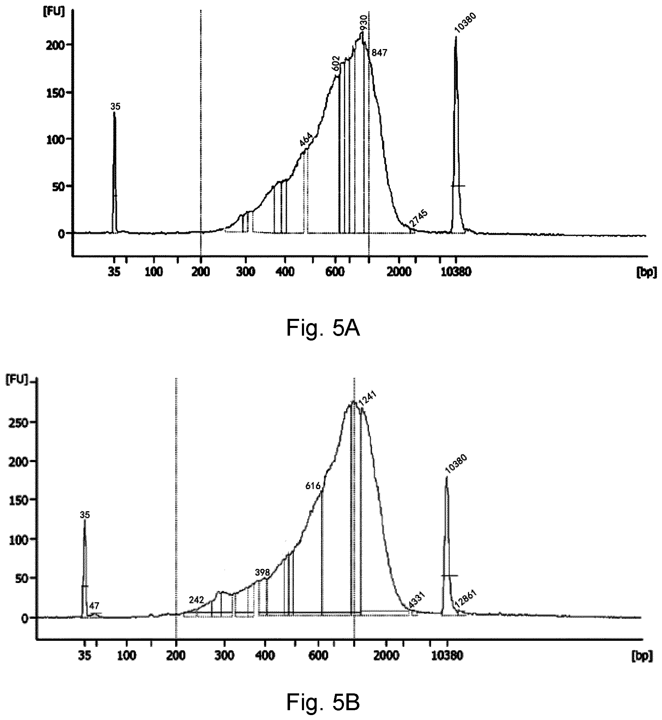

1 Preparation of a PCR system: 42µL of H₂O, 4µL of the 10µM Tn primer (that is, the adapter amplification primer, SEQ ID NO: 2); 4µL of the TSO primer (that is, the SEQ ID NO: 4), and 50µL of 2x KAPA HiFi Hotstart Ready mix.
2 The PCR was performed according to the following conditions: 95 °C, 3 min; 13-20 cycles (98°C, 20s; 58°C, 20s; 72°C, 3 min); 72°C, 5 min; 4°C, keeping the temperature.
3 After the PCR was finished, 60 µL of (0.6X) VAHTSTM DNA Clean Beads (balancing being performed for 30 min at room temperature in advance) was used to purify and recycle a PCR product.
4 The PCR purified product was quantified with a Qubit fluorimeter, and an Agilent 2100 biological analyzer is used for detecting the distribution of fragments (see Figs. 5A and 5B for a cell line sample and a solid tissue sample, respectively).

### (III) cDNA cyclization

A cyclization process was the same as the steps of full-length RNA sequencing.

### (IV) 5'RNA amplification

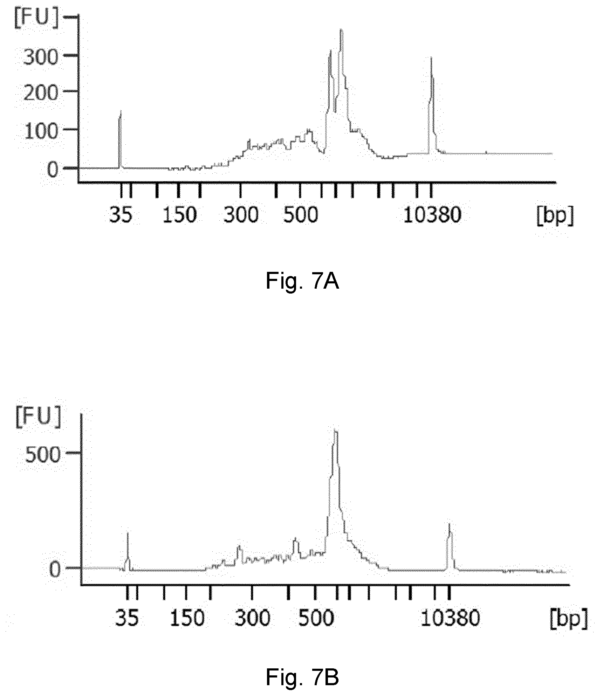

1 The cyclized DNA product starting at 10-50 ng was taken, the volume was made up to 42µL with NF-H₂O; and 4µL of the 20µM poly(A) primer (SEQ ID NO: 6: 5'-phos-AAAAAAAAAAAAAAAAAAAAAAAAAAAAAA-3'), 4µL of a 20µM TCR/BCR primer (10x Genomics V(D) J sequence: PN-1000005, PN-1000016) or the random primer (5'-NNNNNN-3') and 50ul of 2x KAPA HiFi Hotstart Ready mix were added.
2 The PCR was performed according to the following conditions: 95 °C, 3 min; 13-20 cycles (98 °C, 20s; 60 °C, 20s; 72 °C, 30s); 72 °C, 5 min; 4 °C, keeping the temperature.
3 0.5×+1.0× VAHTSTM DNA Clean Beads magnetic beads were used for PCR product purification, Qubit quantification was performed on the screened fragments, and the Agilent 2100 biological analyzer was used for detecting the distribution of fragments (see Figs. 7A and 7B for a cell line sample and a solid tissue sample, respectively, Fig. 7A shows a 2100 detection result for TCR-specific primer amplification, and Fig. 7B shows a 2100 detection result for BCR-specific primer amplification).

### (V) cDNA 5' library construction (the subsequent steps were the same as Embodiment 1 and were not described again).

### Detection and verification:

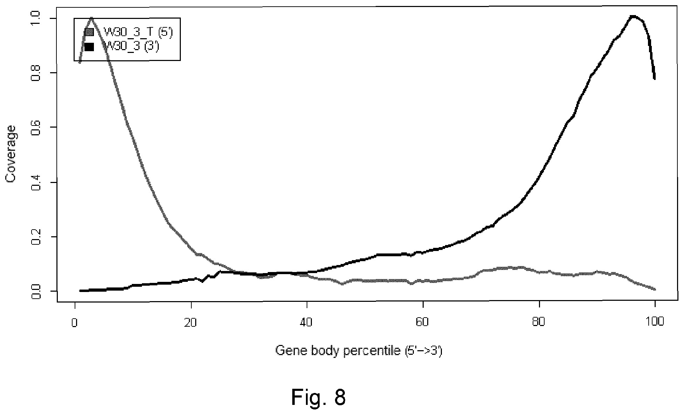

The library constructed in Embodiment 2 was sequenced with a sequencer of an MGI sequencing platform; then the coverage of transcripts was analyzed by 5' terminal and 3' terminal sequencing fragments in sequencing data; and a specific result was shown in Fig. 8. The lighter grayscale corresponded to the coverage of the 5' terminal and the darker grayscale corresponded to the coverage of the 3' terminal. It can be seen, from Fig. 8, that, by means of the method of the present invention, information of the 5' terminal and the 3' terminal of the transcripts can be effectively captured.

The above are only the preferred embodiments of the present invention and are not intended to limit the present invention. For those skilled in the art, the present invention may have various modifications and variations. Any modifications, equivalent replacements, improvements and the like made within the spirit and principle of the present invention all fall within the scope of protection of the present invention.

## Claims

1. A method for constructing an RNA sequencing library, comprising:
acquiring a single-stranded cDNA, which is a reverse transcription product of mRNA, wherein the 3'-terminal of the single-stranded cDNA comprises a cDNA tag sequence; cyclizing the single-stranded cDNA to obtain a single-stranded cyclized cDNA;
amplifying the single-stranded cyclized cDNA with a primer combination, which is formed by a random primer or a gene-specific primer and a cDNA tag primer, so as to obtain an amplified fragment, wherein the cDNA tag primer is at least a part of the cDNA tag sequence; and
performing fragmentation for library construction on the amplified fragment, so as to obtain the RNA sequencing library.

2. The method according to claim 1, wherein the acquiring a single-stranded cDNA, which is a reverse transcription product of mRNA, wherein the 3'-terminal of the single-stranded cDNA comprises a cDNA tag sequence, comprises:
performing reverse transcription on the mRNA, so as to obtain a first strand cDNA;
amplifying the first strand cDNA to obtain a double-stranded cDNA, wherein the 3' terminal of a second strand cDNA, which is complementary to the first strand cDNA, comprises the cDNA tag sequence, and the cDNA tag sequence comprises a poly(A); and
melting the double-stranded cDNA to obtain the single-stranded cDNA.

3. The method according to claim 2, wherein the cDNA tag sequence successively comprises, in a direction from 3' to 5', a second PCR adapter, a second cell barcode, a second Unique Molecular Identifier (UMI) and the poly(A).

4. The method according to claim 1, wherein the mRNA is derived from a single-cell sample, and the mRNA is a single-cell mRNA.

5. The method according to claim 4, wherein preparing the single-cell mRNA with a droplet method, so as to make the single-cell mRNA ligated to a solid support, and preferably, the solid support is a bead.

6. The method according to claim 5, wherein preparing the single-cell mRNA with the droplet method, so as to make the single-cell mRNA ligated to the bead, comprises:
respectively providing a single-cell suspension and the bead, wherein the bead carries a bead tag sequence, and the terminal of the bead tag sequence comprises a poly(dT); and
wrapping the single-cell suspension and the bead into droplets, wherein each droplet comprises one single cell and one bead, and the bead is combined with the poly(A) of the mRNA in the single-cell suspension through the poly(dT), to connect the mRNA in the single-cell suspension to the bead, so as to obtain the single-cell mRNA.

7. The method according to claim 6, wherein the bead tag sequence successively comprises, in a direction from 5' to 3', a first PCR adapter, a first cell barcode, a first UMI and the poly(dT); and correspondingly, the cDNA tag sequence successively comprises, in a direction from 3' to 5', a second PCR adapter, a second cell barcode, a second UMI and a poly(A), wherein the second PCR adapter is complementary to the first PCR adapter, the second cell barcode is complementary to the first cell barcode, and the second UMI is complementary to the first UMI.

8. The method according to claim 2, wherein the 5' terminal of the single-stranded cDNA comprises a sequence of a TSO primer.

9. The method according to claim 8, wherein reverse transcription is performed on the mRNA with a reverse transcriptase and a TSO adapter, so as to obtain the first strand cDNA, wherein the reverse transcriptase has a terminal transferase activity, and the 3' terminal of the first strand cDNA comprises a complementary sequence of the TSO adapter; and
amplifying the first strand cDNA to obtain the second strand cDNA, and the 5' terminal of the second strand cDNA comprises the sequence of the TSO primer.

10. The method according to claim 9, wherein the sequence of the TSO adapter is SEQ ID NO: 1.

11. The method according to claim 9, wherein the reverse transcriptase is selected from an Alpha reverse transcriptase of MGI, a Superscript^{™}II reverse transcriptase of Invitrogen, Superscript IV of Thermo, or Maxima H Minus of Thermo.

12. The method according to claim 2, wherein random amplification and/or full-length amplification is performed on the first strand cDNA, so as to obtain the double-stranded cDNA.

13. The method according to claim 12, wherein amplifying the first strand cDNA with an adapter amplification primer and a TSO primer, so as to obtain the double-stranded cDNA; or
amplifying the first strand cDNA with the adapter amplification primer, a TSO-random primer and the TSO primer, so as to obtain the double-stranded cDNA.

14. The method according to claim 13, wherein the sequence of the adapter amplification primer is SEQ ID NO: 2; the sequence of the TSO primer is SEQ ID NO: 3; and the sequence of the TSO-random primer is SEQ ID NO: 4.

15. The method according to any of claims 1 to 14, wherein cyclizing the single-stranded cDNA to obtain a single-stranded cyclized cDNA, comprises:
ligating the single-stranded cDNA into a ring under the action of a cyclization auxiliary sequence and a ligase, so as to obtain a ligated product; and
performing enzyme digestion on the ligated product to digest the single-stranded cDNA, which is not ligated into the ring, so as to obtain the single-stranded cyclized cDNA, wherein
the cyclization auxiliary sequence is complementary to sequences on two terminals of the single-stranded cDNA.

16. The method according to claim 15, wherein the cyclization auxiliary sequence is selected from SEQ ID NO: 5.

17. The method according to claim 1, wherein the gene-specific primer is a TCR primer for TCR gene amplification and/or a BCR primer for BCR gene amplification.

18. The method according to claim 1, wherein the cDNA tag primer is a poly(A) primer, preferably SEQ ID NO: 6.

19. The method according to claim 1, wherein the performing fragmentation for library construction on the amplified fragment, so as to obtain an RNA sequencing library, comprises:
adding a library adapter to the amplified fragment, so as to obtain the RNA sequencing library.

20. The method according to claim 19, wherein fragmentation with enzyme digestion is performed on the amplified fragment, so as to obtain digested fragments; and
terminal repair, A tailing addition and library adapter ligation are successively performed on the digested fragments, so as to obtain the RNA sequencing library.

21. The method according to claim 19 or 20, wherein after library adapter ligation is performed, the method further comprises performing PCR amplification on the ligated product of the library adapter, so as to obtain the RNA sequencing library.

22. The method according to claim 19, wherein the library adapter is an adapter of an MGI sequencing platform or an adapter of an Illumina sequencing platform.

23. A kit for an RNA library construction, comprising: a cyclization auxiliary sequence, a DNA ligase, a cDNA tag primer, and at least one of the following primers: (a) a random primer; (b) a TCR primer; or (c) a BCR primer.

24. The kit according to claim 23, further comprising an RNA reverse transcription reagent.

25. The kit according to claim 24, wherein the RNA reverse transcription reagent comprises a reverse transcriptase; and the reverse transcriptase is a reverse transcriptase having terminal transferase activity.

26. The kit according to claim 25, wherein the reverse transcriptase is selected from an Alpha reverse transcriptase of MGI, a Superscript^{™}II reverse transcriptase of Invitrogen, Superscript IV of Thermo, or Maxima H Minus of Thermo.

27. The kit according to claim 25, wherein the RNA reverse transcription reagent further comprises a TSO adapter.

28. The kit according to claim 27, wherein the sequence of the TSO adapter is SEQ ID NO: 1.

29. The kit according to claim 23, further comprising a TSO primer and an adapter amplification primer.

30. The kit according to claim 29, wherein the sequence of the adapter amplification primer is SEQ ID NO: 2; and the sequence of the TSO primer is SEQ ID NO: 3.

31. The kit according to claim 29, further comprising a TSO-random primer.

32. The kit according to claim 31, wherein the sequence of the TSO-random primer is SEQ ID NO:4.

33. The kit according to claim 23, wherein the cyclization auxiliary sequence is SEQ ID NO: 5.

34. The kit according to claim 23, wherein the cDNA tag primer is a poly(A) primer.

35. The kit according to claim 34, wherein a sequence of the cDNA tag primer is SEQ ID NO: 6.

36. The kit according to claim 23, further comprising at least one of an exonuclease or a library adapter.

37. The kit according to claim 36, wherein the exonuclease is selected from an exonuclease I or exonuclease III.

38. The kit according to claim 36, wherein the library adapter is an adapter of an MGI sequencing platform or an adapter of an Illumina sequencing platform.

39. The kit according to claim 38, wherein the MGI sequencing platform is selected from a bubble adapter; and an adapter of the Illumina sequencing platform is selected from P5 and P7 adapters.

40. The kit according to claim 23, wherein the DNA ligase is selected from a T4 DNA ligase.

41. The kit according to claim 23, further comprising a solid support, wherein the solid support is provided with a support tag sequence; the cDNA tag primer is complementary to at least a part of the support tag sequence; and preferably, the solid support is a bead, and the support tag sequence is a bead tag sequence.

42. The kit according to claim 41, wherein the support tag sequence successively comprises, in a direction from 5' to 3', a first PCR adapter, a first cell barcode, a first UMI and the poly(dT).

43. A method for sequencing an RNA library, comprising:
constructing an RNA sequencing library with the method for constructing an RNA sequencing library according to any of claims 1 to 22, and
performing sequencing on the RNA sequencing library.
